# EUROPÄISCHE PATENTANMELDUNG

(11) **EP 2 664 271 A1**
(43) Veröffentlichungstag der Anmeldung: **20.11.2013**
(21) Anmeldenummer: 13165720.7
(22) Anmeldetag: 29.04.2013
(51) Int. Cl.: A61B 1/04, A61B 1/045, A61B 1/00, A61B 1/06, A61B 1/227, G06F 1/32

(54) **Videoendoskop mit einem Beschleunigungssensor**

(30) Priorität: 16.05.2012 DE 102012009749
(71) Anmelder: Karl Storz GmbH & Co. KG, 78532 Tuttlingen (DE)
(72) Erfinder: Weiß, Stefan, 78532 Tuttlingen (DE)
(74) Vertreter: Witte, Weller & Partner

(57) **Zusammenfassung**

Ein Videoendoskop (10) weist einen Lichtleiter (40) auf, der von einer Lichtquelle (36) mit Beleuchtungslicht versorgbar ist. Ferner ist ein Bildaufnahmesystem (28) mit einem Linsensystem (30) und einem elektrisch versorgten Videochip (32) vorhanden. Ein Beschleunigungssensor (50) detektiert eine Bewegung oder Nichtbewegung des Videoendoskops, wodurch die Lichtquelle (36) und/oder der elektrische Versorgung des Videochips (32) zumindest in einen energieverbrauchsarmen Stand-by-Modus versetzbar ist. Es wird vorgeschlagen eine Zeitmesseinrichtung (54) vorzusehen, die bei einer Nichtbewegung des Videoendoskops (10) erst nach einer bestimmten gemessenen Zeitspanne ein Versetzen in den Stand-by-Modus ermöglicht (Fig. 2).

## Beschreibung

Die Erfindung betrifft ein Videoendoskop, mit einem Lichtleiter, der von einer Lichtquelle mit Beleuchtungslicht versorgbar ist, mit einem Bildaufnahmesystem, das ein Linsensystem und einen elektrisch versorgten Videochip aufweist, und mit einem Beschleunigungssensor, der eine Bewegung oder Nichtbewegung des Videoendoskops detektiert, und der bei Nichtbewegung des Videoendoskops die Lichtquelle und/oder die elektrische Versorgung des Videochips zumindest in einen energieverbrauchsarmen Stand-by-Modus versetzt.

Ein derartiges Videoendoskop ist aus der DE 10 2009 043 652 A1 bekannt.

Derartige Videoendoskope weisen eine Lichtquelle insbesondere in Form einer LED auf, die üblicherweise von Hand ein- und ausgeschaltet werden kann. Zusätzlich ist ein Bildaufnahmesystem enthalten, das ein Linsensystem und einen elektrisch versorgten Videochip aufweist. Dadurch kann die üblicherweise am distalen Ende eintretende Bildinformation über das Linsensystem einem hochauflösenden Videochip zugeführt werden. Dort wird das Bildsignal in elektrische Signale umgewandelt, die meist distalseitig über ein Kabel abgeführt und einem Monitor zur Visualisierung zugeführt werden.

Das eingangs genannte Videoendoskop weist zumindest ein beim Betrieb des Instruments sich erwärmendes Bauteil auf und ist mit einem Sensor zum selbstständigen Erfassen des Gebrauchs- oder Nichtgebrauchszustandes des Instruments ausgestattet. Es ist eine Steuerung vorhanden, die während des Nichtgebrauchs des Instruments das zumindest eine beim Betrieb des Instruments sich erwärmende Bauteil leistungsreduziert ansteuert oder abschaltet und während des Gebrauchs des Instruments das sich erwärmende Bauteil mit der für den Gebrauch vorgesehenen Leistung ansteuert oder einschaltet.

Als Sensoren eignen sich zwei- oder dreidimensionale Lage- bzw. Beschleunigungssensoren. Diese erfassen jegliche Lageveränderung des Videoendoskops. Lageveränderung bedeutet dabei Bewegung, auch wenn dies nur linear längs einer Achse erfolgt, Drehen oder Kippen und dgl.

Liegen keine solchen Lageveränderungen vor, wird das von dem Sensor detektiert und insbesondere das Wärme erzeugende Bauteil, nämlich die Lichtquelle, wird abgeschaltet.

Das hat zum einen den Sinn, das Gerät bei Nichtgebrauch in einen energiesparenden Stand-by-Modus zu versetzen. Zum anderen soll dadurch vermieden werden, dass sich das Gerät durch das Wärme abstrahlende Bauelement stark erwärmt.

Bei der eingangs genannten Vorrichtung ist der Sensor so ausgestaltet, dass er den Nichtgebrauch des Gerätes in einer ganz bestimmten Stellung detektiert, in der das Videoendoskop in eine Halterung eingeschoben ist.

Nachteilig daran ist, dass dazu das Videoendoskop in eine spezielle Halterung eingeschoben werden muss. Eine solche Halterung stellt oftmals eine Einschränkung des Handhabungsbereiches des Arztes dar, der mit dem Videoendoskop eine Untersuchung durchführen möchte.

Ferner wird bei zahlreichen Untersuchungen das Videoendoskop in periodischen Abständen benutzt, abgelegt, wieder benutzt, wieder abgelegt, wobei das Ablegen der behandelnde Arzt meist in seiner unmittelbaren Nähe durchführen möchte.

Ist beispielsweise das Videoendoskop ein Otoskop, das zur Untersuchung der Ohrmuschel, des Gehörganges und des Trommelfells dient, spielen sich oftmals die nachfolgenden Vorgänge ab. Zunächst wird das Videoendoskop vom Arzt herangeschafft und eingeschaltet und in den Gehörgang eingeschoben, um eine erste Inspektion durchzuführen. Dann wird das Gerät zur Seite gelegt, um in dem Gehörgang gewisse Manipulationen durchzuführen, beispielsweise um ein Mittel einzutropfen, das Verstopfungen lösen oder beseitigen soll. Danach wird erneut das Otoskop angesetzt, um zu überprüfen, ob auch alle Stellen erreicht worden sind. Dann wird nach einer gewissen Einwirkzeit, meist mit einem Stäbchen oder dgl., eine Reinigung des Gehörganges durchgeführt. Danach muss erneut das Otoskop angesetzt werden, um den Erfolg dieses Vorganges zu überprüfen. War dieser noch nicht erfolgreich, muss das wiederholt werden.

Abschließend wird manchmal ein Gewebestreifen mit einem Arzneimittel in den Gehörgang eingesetzt, wenn Entzündungen zu befürchten sind. Abschließend wird nochmals das Otoskop zur Lageüberprüfung angesetzt.

Wenn wie in der eingangs genannten Vorrichtung dazu jedes Mal das Gerät in eine Halterung eingesetzt werden muss, ist das umständlich.

Wenn diese Vorgänge relativ rasch hintereinander stattfinden, muss permanent das Gerät ein- bzw. ausgeschaltet werden, was der Lebensdauer nicht zuträglich ist. Das nur kurzzeitige Ein- und Ausschalten, insbesondere der Lichtquelle, ist sowohl für den Arzt als auch möglicherweise für den Patienten störend, wenn der Arzt das Videoendoskop unmittelbar nahe dem Patienten ablegt. Da es sich oftmals um sehr lichtstarke Geräte handelt, kann es sehr störend, insbesondere vom Patienten, empfunden werden, wenn eine solche Lichtquelle in seiner unmittelbaren Nähe quasi im Sekundentakt an- und ausgeht.

Es ist daher Aufgabe der Erfindung, hier Abhilfe zu schaffen und den Ein- und Abschaltevorgang zu vereinfachen und den jeweiligen Untersuchungen mehr anpassbar zu machen.

Erfindungsgemäß wird die Aufgabe dadurch gelöst, dass eine Zeitmesseinrichtung vorhanden ist, die bei einer detektierten Nichtbewegung des Videoendoskops erst nach einer bestimmten gemessenen Zeitspanne ein Versetzen in den Stand-by-Modus ermöglicht.

Diese Maßnahme hat den Vorteil, dass beim Detektieren der Nichtbewegung durch den Sensor zunächst über die Zeitmesseinrichtung eine Zeitnahme bzw. eine Zeitmessung gestartet wird und erst nach deren Ablauf in den Stand-by-Modus geschaltet wird. Unter Stand-by-Modus kann sowohl ein völliges Abschalten oder nur ein Verdunkeln der LED bzw. ein Unterbrechen oder Abschalten des Videosignals verstanden werden.

Greift man auf das Beispiel des Otoskops zurück, so ist die Zeitspanne zwischen den Vorgängen, bei denen das zuvor erwähnte Videoendoskop benutzt wird, relativ ähnlich und liegt im Bereich von mehreren Sekunden, meist unter einer Minute.

Es ist nun möglich, durch die Zeitmesseinrichtung eine bestimmte Zeitspanne vorzusehen, innerhalb derer das Gerät noch nicht in den Stand-by-Modus versetzt wird, sondern erst nach Ablauf dieser Zeitspanne.

Dabei kann eine solche Zeitspanne ausgewählt bzw. eingestellt werden, die die relativ kurzen Pausen zwischen üblichen Handhabungen überbrückt, so dass das Gerät nicht sofort in den Stand-by-Modus versetzt wird. Sinnvollerweise wird man die Zeitspanne dann so wählen, dass sie auf jeden Fall so lang ist wie der Zeitraum zwischen zwei üblichen Handhabungen innerhalb einer Behandlung bzw. Untersuchung.

Benutzt der Arzt das Gerät erstmals, findet ja eine Bewegung statt und die Lichtquelle und/oder der elektrisch versorgte Videochip werden versorgt. Soll nun das Gerät nur für eine kurze Zeit beiseitegelegt werden, um beispielsweise in den Gehörgang einige Tropfen einzubringen und danach das Gerät erneut eingesetzt werden soll, schaltet das Gerät noch nicht in den Stand-by-Modus. Erst nach Überschreiten der vorgegebenen Zeitspanne geht es in diesen Modus über.

Beträgt erfahrungsgemäß bei einer solchen Untersuchung die Zeitspanne etwa 30 Sekunden, so kann man die Zeitmesseinrichtung so einstellen, dass sie 40 Sekunden läuft und erst danach die energieverbrauchenden Bauteile, also die Lichtquelle und den Videochip, in den Stand-by-Modus versetzt.

Ist das Videoendoskop allerdings dann für mehr als 40 Sekunden beiseite gelegt, weil beispielsweise der Arzt längerfristige Manipulationen vornimmt oder ein Gespräch mit dem Patienten führt, schaltet das Gerät ab. Wird es dann wieder ergriffen, detektiert das der Sensor und die Energieverbraucher werden dann wieder elektrisch versorgt.

Die jeweilige Zeitmessung der Zeitmesseinrichtung startet immer dann, wenn der Sensor den Status "Nichtbewegung" detektiert. Sobald der Sensor den Status "Bewegung" detektiert, werden die Energieverbraucher versorgt bzw. bleiben versorgt, auch wenn die zuvor erwähnte Zeitspanne noch nicht abgelaufen ist.

In einer weiteren Ausgestaltung der Erfindung ist der Beschleunigungssensor ein 3-Achsen-Beschleunigungssensor.

Diese Maßnahme hat den Vorteil, dass ein solcher Sensor ein kostengünstig erhältlicher und ein sehr empfindlicher Sensor ist.

In einer weiteren Ausgestaltung der Erfindung ist der Beschleunigungssensor auf einem Flexprint angebracht.

Diese Maßnahme hat den Vorteil, dass der Sensor direkt auf der Platine montiert ist, an der auch die anderen für die Steuerung notwendigen Bauteile wie Prozessoren, Schaltkreise und dgl. vorhanden sind. Bei der Herstellung kann dann das Flexprint entsprechend vormontiert und konfiguriert werden. Wie gesagt sind moderne 3-Achsen-Beschleunigungssensoren so hochempfindlich, dass sie nicht an besonders exponierten Stellen angebracht werden, die bei Bewegung einen möglichst großen Weg durchführen, beispielsweise am vorderen oder hinteren Ende eines Geräts, das bei Kippbewegungen ja die größten Bewegungsstrecken durchführt. Das trägt erheblich zu einer einfachen und vorfertigbaren Konstruktion bei.

In einer weiteren Ausgestaltung der Erfindung ist die gemessene Zeitspanne der Zeitmesseinrichtung herstellerseits einstellbar.

Wird ein bestimmter Typ eines Videoendoskops üblicherweise bei Routineuntersuchungen eingesetzt, bei denen das Videoendoskop mehrfach und in relativ kurzen Abständen zwischen dem Zustand "Bewegung" und "Nichtbewegung" versetzt wird und sind diese Zeitspannen bekannt, kann herstellerseits die Zeitmesseinrichtung so eingestellt werden, dass solche Zeitspannen überbrückt werden können.

Das heißt, das Gerät schaltet zumindest einen oder alle Energieverbraucher erst nach dieser voreingestellten Zeitspanne ab.

Das eröffnet auch die Möglichkeit, nur einzelne, besonders störende Energieverbraucher bei Detektieren von "Nichtbewegung" nach einer relativ kurzen Zeitspanne abzuschalten, beispielsweise eine extrem helle und wärmeerzeugende Lichtquelle. Andere Stromverbraucher können erst später oder überhaupt nicht abgeschaltet werden, beispielsweise der Videochip, der das Bild umsetzt. Es können auch alle Stromverbraucher gleichzeitig abgeschaltet werden, d.h. die Lichtquelle wird abgeschaltet und der Videochip bzw. der damit verbundene Monitor wird dann so geschaltet, dass kein Bild ersichtlich ist.

Das erhöht die Flexibilität des Gerätes im Hinblick auf die Handhabung und insbesondere den Wunsch der Handhabungsperson, welche Energieverbraucher zu welchem Zeitpunkt und ob überhaupt abgeschaltet werden sollen oder nicht.

In einer weiteren Ausgestaltung der Erfindung ist die gemessene Zeitspanne vom Benutzer am Videoendoskop über einen Regler einstellbar.

Diese Maßnahme hat den Vorteil, dass die Handhabbarkeit und Individualität noch zusätzlich dadurch erhöht wird, dass der Handhabungsperson die Möglichkeit gegeben wird, diese Zeitspanne der Zeitmesseinrichtung selbst einzustellen.

Dann sind entsprechende schaltungstechnische Maßnahmen und Schalter vorgesehen, die der Handhabungsperson ermöglichen, diese Zeitspanne zu verlängern oder zu verkürzen.

Es versteht sich, dass die vorstehend genannten und die nachstehend noch zu erläuternden Merkmale nicht nur in den angegebenen Kombinationen, sondern auch in anderen Kombinationen einsetzbar sind, ohne den Rahmen der vorliegenden Erfindung zu verlassen.

Die Erfindung wird nachfolgend anhand der beiliegenden Zeichnungen näher beschrieben und erläutert. Es zeigen:
- Fig. 1: perspektivisch eine Ansicht eines Videoendoskops in Form eines Otoskops, und
- Fig. 2: einen Längsschnitt des Videoendoskops von Fig. 1.

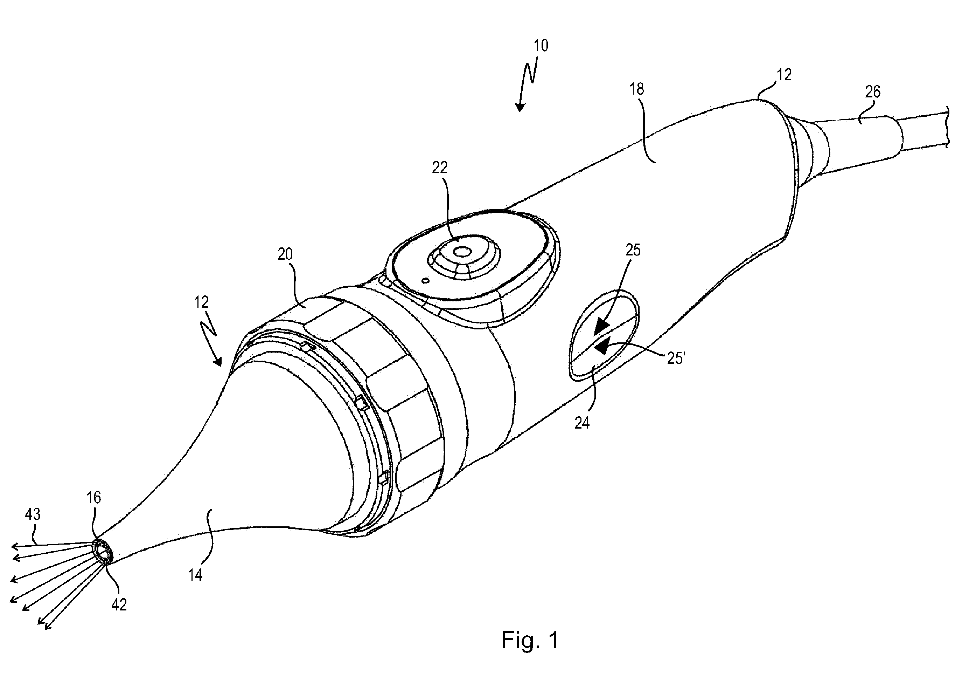

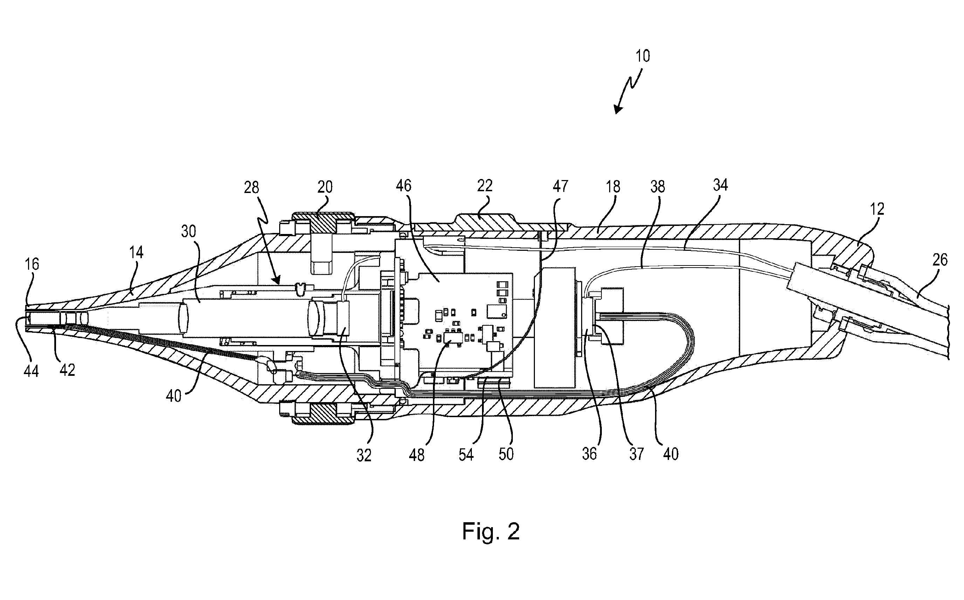

Ein in den Fig. 1 und 2 dargestelltes Videoendoskop ist in seiner Gesamtheit mit der Bezugsziffer 10 bezeichnet.

Im dargestellten Ausführungsbeispiel ist das Videoendoskop 10 ein Otoskop. Otoskope dienen Untersuchungen am gesamten äußeren Ohr. Dazu gehören die Ohrmuschel, der Gehörgang und das Trommelfell.

Das Videoendoskop 10 weist ein Gehäuse 12 auf, das distalseitig einen sich zum distalen Ende 16 hin stark verjüngenden trichterförmigen Abschnitt 14 aufweist. Der Durchmesser im Bereich des distalen Endes 16 ist so gering, dass dieses distale Ende in ein Ohr eingeführt und auch relativ weit in den Gehörgang bis nahe an das Trommelfell eingeschoben werden kann.

Proximalseitig des trichterförmigen Abschnitts 14 ist das Gehäuse 12 als ein etwa stabförmiger Handgriff 18 ausgebildet, über den das Videoendoskop 10 von der Hand eines Menschen ergriffen werden kann.

Im Übergang zwischen trichterförmigem Abschnitt 14 und Handgriff 18 ist ein drehbarer Fokussierring 20 einer Fokussiereinrichtung vorhanden, dessen Funktionsweise später beschrieben wird.

Ferner ist am Handgriff 18 proximalseitig vom Fokussierring 20 ein Ein/Aus-Schalter 22 angeordnet. Über diesen Ein/Aus-Schalter 22 kann das Videogerät für einen Betrieb eingeschaltet und nach einem Einsatz beispielsweise zum Verstauen oder für eine Reinigung ausgeschaltet werden.

Aus Fig. 1 ist ersichtlich, dass, wenn der Handgriff 18 von einer Hand ergriffen ist, der Fokussierring 20 durch zwei Finger, beispielsweise den Daumen und den Zeigefinger, gedreht werden kann. Der Ein/Aus-Schalter 22, der als Druckschalter ausgebildet ist, kann durch den Daumen betätigt werden.

In einem seitlichen Bereich ist am Handgriff 18 noch ein Regler 24 angeordnet, der mit einer Zeitmesseinrichtung schaltungstechnisch verbunden ist. Der Regler 24 weist zwei Schaltelemente 25, 25' auf. Das Schaltelement 25 ist mit einem in der Darstellung von Fig. 1 nach oben weisenden Pfeil versehen, das darunterliegende Schaltelement 25' mit einem nach unten gerichteten Pfeil.

Durch Betätigung dieser Schaltelemente kann die Zeitspanne einer nachfolgend zu beschreibenden Zeitmesseinrichtung erhöht oder erniedrigt werden, also geregelt werden. Dabei dient das Schaltelement 25 zur Verlängerung der Zeitspanne und das Schaltelement 25' zur Verkürzung der Zeitspanne.

Im Innern des Videoendoskops 10 ist, wie das insbesondere aus der Schnittdarstellung von Fig. 2 ersichtlich ist, im Bereich des trichterförmigen Abschnitts 14 ein Bildaufnahmesystem 28 aufgenommen. Das Bildaufnahmesystem 28 weist ein Linsensystem 30 auf, das sich bis zu dem distalen Ende des trichterförmigen Abschnitts 14 erstreckt und dort über ein Abschlussfenster 44 abgeschlossen ist.

Über das Linsensystem 30 wird in den trichterförmigen Abschnitt 14 eintretendes Licht entsprechend optisch bearbeitet und einem am proximalen Ende des Linsensystems 30 angeordneten Videochip 32 zugeführt. Der Videochip 32 ist ein hochauflösender CMOS-Kamerachip.

Dieser Videochip 32 wird über eine Leitung 34 elektrisch versorgt. Über diese Leitung wird auch die vom Videochip 32 in elektrische Signale umgewandelte Bildinformation des Linsensystems 30 abgeführt, und zwar durch eine am proximalen Ende des Gehäuses 12 eingesetzte Kabeltülle 26. Die Leitung 34 führt einerseits zu einem Stromversorger und auch zu einer Vorrichtung zur Verarbeitung der Bildinformation und ggf. zur Visualisierung auf einem Monitor.

Unmittelbar proximal vom Videochip 32 und mit diesem verbunden ist ein Flexprint 46, das die ganzen Steuerungs- und Verarbeitungsprozesse durchführt. Der Videochip 32 ist über ein nicht näher dargestelltes Interface mit dem Flexprint 46 verbunden.

Proximalseitig des Flexprints 46 ist eine von diesem abgeschirmte Lichtquelle 36 in Form einer leistungsstarken LED 37 angeordnet. Wie ersichtlich strahlt die Lichtquelle 36 nach proximal ab. Diese ist über ein Stromkabel 38, das ebenfalls durch die Kabeltülle 26 geführt wird, mit einer Energiequelle verbunden.

Die nach proximal abstrahlende LED 37 speist ihr Licht in einen flexiblen Lichtleiter 40 ein, der aus einem Bündel von flexiblen Glasfasern besteht, die in einer Hülle aufgenommen sind. Der Lichtleiter 40 wird über eine Krümmung an der Innenseite des Gehäuses 12 und des trichterförmigen Abschnitts 14 bis zum distalen Ende geführt und spleißt dort in einen Ring 42 auf. Das heißt, die Lichtfasern umgeben in diesem Bereich das Linsensystem 30 umfänglich.

Dadurch wird, wie das insbesondere aus Fig. 1 ersichtlich ist, gleichmäßig um das Abschlussfenster 44 herum Beleuchtungslicht abgestrahlt.

Wie aus der Schnittdarstellung von Fig. 2 zu entnehmen, ist das Flexprint 46 über ein Kabel 47 mit dem Ein/Aus-Schalter 22 verbunden.

Am Flexprint 46 ist ein 3-Achsen-Beschleunigungssensor 50 montiert, in dem eine Zeitmesseinrichtung 54 integriert ist. Die Zeitmesseinrichtung 54 ist mit dem Regler 24 verbunden.

Durch den Ein/Aus-Schalter 22 wird das Videoendoskop 10 betriebsbereit gemacht bzw. abgeschaltet, wenn überhaupt kein Betrieb gewünscht ist, beispielsweise beim Verstauen oder zum Reinigen oder Desinfizieren des Geräts.

Der Ein/Aus-Schalter 22 steuert somit die grundsätzliche Betriebsbereitschaft.

Dadurch wird die Lichtquelle 36 aktiviert und über den Lichtleiter 40 wird Beleuchtungslicht abgestrahlt, wie das in Fig. 1 durch die Pfeile 43 angedeutet wird. Der Videochip 32 ist ebenfalls betriebsbereit und kann ein digitales Informationssignal abgeben, das entweder in einer Einheit gespeichert oder nach Verarbeiten auf einem Monitor visualisiert wird.

Das Linsensystem 30 kann zur Fokussierung über den Fokussierring verstellt werden. Dies dient nicht nur allein einer "Scharfstellung" des Bildes, sondern kann zusätzlich zwischen einem Nah- und Fernbereich fokussieren.

Bei einem Otoskop ist es beispielsweise wünschenswert, beim Ansetzen an der Ohrmuschel zunächst den gesamten Gehörgang zu überblicken, was einem Fernbereich entsprechen würde. Wird das Otoskop bis unmittelbar an das Trommelfell herangeführt, kann es auf den Nahbereich geschaltet werden, um jeweils ein scharfes Bild zu erzeugen.

Der 3-Achsen-Beschleunigungssensor 50 erfasst bei eingeschaltetem Videoendoskop 10 die Zustände "Bewegung" oder "Nichtbewegung". Wird das Videoendoskop 10 bewegt, wird im vorliegenden Fall sowohl die Lichtquelle 36 geschaltet, sie leuchtet also, und auch der Videochip 32 ist aktiviert, um ein Bild zu erzeugen.

Wird das Videoendoskop 10 nicht mehr bewegt, detektiert der 3-Achsen-Beschleunigungssensor 50 den Zustand "Nichtbewegung". Nach dem Detektieren löst dieser ein Signal an der Zeitmesseinrichtung 54 dahingehend aus, dass nun eine bestimmte Zeitspanne läuft. Nach Ablauf dieser Zeitspanne wird über die Prozessoren 48 des Flexprints 46 in einen Stand-by-Modus geschaltet.

In diesem Stand-by-Modus wird die Lichtquelle 36 abgeschaltet. Das vom Videochip 32 ausgesendete Videosignal wird unterbrochen und es wird ein schwarzer Bildschirm angezeigt.

Sobald das Videoendoskop 10 wieder bewegt wird, selbst dann, wenn die Zeitspanne noch nicht abgelaufen sein sollte, schaltet die Lichtquelle 36 wieder ein und das Videobild wird wieder angezeigt.

Die Dauer der Zeitspanne, die von der Zeitmesseinrichtung 54 gemessen wird, kann herstellerseits eingestellt werden.

Es kann am Gehäuse 12 auch eine USB-Schnittstelle oder dgl. vorgesehen sein, über die bei Revisionen oder auf Wunsch der Handhabungsperson diese Zeitspanne eingestellt oder verändert werden kann.

Im dargestellten Ausführungsbeispiel besteht über den Regler 24 noch die Möglichkeit, dass die Handhabungsperson selbst individuell diese vorgegebene Zeitspanne verändern kann, sprich entweder verlängern oder verkürzen.

Wenn Routineuntersuchungen durchgeführt werden, bei denen das Otoskop mehrfach an das Ohr angesetzt wird und die Abstände dazwischen üblicherweise im Bereich von 30 Sekunden liegen, kann beispielsweise diese Zeitspanne auf 40 Sekunden eingestellt werden. Für andere Untersuchungen kann die Zeitspanne über den Regler 24 verändert werden.

Wird nach Erfassen des Zustandes "Nichtbewegung" das Videoendoskop 10 schon etwa nach 30 Sekunden ergriffen, also bewegt, ist es noch nicht in den Stand-by-Modus übergegangen und schaltet auch nicht in diesen Modus.

Erst wenn diese Zeitspanne abgelaufen ist und nach wie vor der Zustand "Nichtbewegung" detektiert wird, wird das Gerät in den Stand-by-Modus geschaltet. Wird es dann ergriffen, wird es sofort wieder aktiviert, d.h. die Lichtquelle 36 angeschaltet und der Videochip 32 aktiviert.

Schaltungstechnisch kann es auch so vorgesehen sein, dass nur eines der beiden stromversorgten Geräte, also entweder die Lichtquelle oder der Videochip, entsprechend geschaltet werden, wobei es meist Sinn macht, auf jeden Fall die Lichtquelle 36 zu- oder abzuschalten, da dort der Energieverbrauch und auch möglicherweise die Erwärmung wesentlich stärker ausgeprägt ist und der Videochip ohne Beleuchtung ein schwaches Bild erzeugt.

## Patentansprüche

1. Videoendoskop, mit einem Lichtleiter (40), der von einer Lichtquelle (36) mit Beleuchtungslicht versorgbar ist, mit einem Bildaufnahmesystem (28), das ein Linsensystem (30) und einen elektrisch versorgten Videochip (32) aufweist, und mit einem Beschleunigungssensor (50), der eine Bewegung oder Nichtbewegung des Videoendoskops (10) detektiert und der bei Nichtbewegung des Videoendoskops (10) die Lichtquelle (36) und/oder die elektrische Versorgung des Videochips (32) zumindest in einen energieverbrauchsarmen Stand-by-Modus versetzt, **dadurch gekennzeichnet, dass** eine Zeitmesseinrichtung (54) vorhanden ist, die bei einer detektierten Nichtbewegung des Videoendoskops (10) erst nach einer bestimmten gemessenen Zeitspanne ein Versetzen in den Stand-by-Modus ermöglicht.

2. Videoendoskop nach Anspruch 1, **dadurch gekennzeichnet, dass** die Lichtquelle (36) eine LED (37) aufweist.

3. Videoendoskop nach Anspruch 1 oder 2, **dadurch gekennzeichnet, dass** der Beschleunigungssensor (50) ein 3-Achsen-Beschleunigungssensor ist.

4. Videoendoskop nach Anspruch 1 oder 2, **dadurch gekennzeichnet, dass** der Beschleunigungssensor (50) auf einem Flexprint (40) angebracht ist.

5. Videoendoskop nach einem der Ansprüche 1 bis 4, **dadurch gekennzeichnet, dass** die gemessene Zeitspanne der Zeitmesseinrichtung (54) herstellerseits einstellbar ist.

6. Videoendoskop nach einem der Ansprüche 1 bis 5, **dadurch gekennzeichnet, dass** die gemessene Zeitspanne der Zeitmesseinrichtung (54) vom Benutzer am Videoendoskop (10) über einen Regler (24) einstellbar ist.

7. Verfahren zum Einschalten oder Ausschalten eines Videoendoskops (10), das mit einer Lichtquelle (36), einem elektrisch versorgbaren Videochip (32) und einem Beschleunigungssensor (50) versehen ist, der bei Nichtbewegung des Videoendoskops (10) die elektrische Versorgung der Lichtquelle (36) und/oder des Videochips (32) zumindest stark reduziert, **dadurch gekennzeichnet, dass** mittels einer Zeitmesseinrichtung (54) eine Zeitspanne nach Nichtbewegung des Videoendoskops (10) gemessen wird und dass die Lichtquelle (36) und/oder der Videochip (32) erst nach Ablauf dieser Zeitspanne in den energieverbrauchsarmen Stand-by-Modus versetzt wird.
